# EUROPEAN PATENT SPECIFICATION

(11) **EP 0 725 617 B1**
(45) Date of publication and mention of the grant of the patent: **23.04.2003**
(21) Application number: 94930474.5
(22) Date of filing: 26.09.1994
(51) Int. Cl.: A61F 13/58, C09J 7/02

(54) **STRUCTURED ADHESIVE CLOSURE SYSTEMS**
STRUKTURIERTER KLEBEVERSCHLUSS
SYSTEMES DE FERMETURE ADHESIFS STRUCTURES

(30) Priority: 29.10.1993 US 145341
(43) Date of publication of application: 14.08.1996
(73) Proprietor: MINNESOTA MINING AND MANUFACTURING COMPANY, St. Paul, Minnesota 55133-3427 (US)
(72) Inventor: BOYER, Charles E., III, Oakdale, MN 55128 (US); RASMUSSEN, David P., Woodbury, MN 55125 (US); SETH, Jayshree, Woodbury, MN 55125 (US); SIPINEN, Alan J., North Oaks, MN 55127 (US); UNRUH, William C., Inver Grove Heights, MN 55077 (US); VELASQUEZ UREY, Ruben Emilio, Lake Elmo, MN 55042 (US)
(74) Representative: VOSSIUS & PARTNER
(86) International application number: US9410837
(87) International publication number: WO95011655

(56) References cited:
- EP-A- 0 316 601
- EP-A- 0 336 639
- EP-A- 0 418 954
- WO-A-93/23488
- DE-A- 4 202 704
- DE-U- 8 414 618
- US-A- 3 630 201
- US-A- 4 023 570

## Description

The invention relates to a closure system formed with two opposing surfaces that are adhered together using a pressure-sensitive adhesive layer to form a closure such as is used in an incontinent article or diaper. Further, the invention relates to an improved pressure-sensitive adhesive tape for use in such a closure system where the pressure-sensitive adhesive coating of the tape has a microstructure providing improved or controlled peel performance.

Closure systems, such as used in incontinent articles or diapers are conventionally provided with a pressure-sensitive adhesive (PSA) coated tape tab acting as a fastener. This tape tab fastener is designed to adhere at two opposing ends to two opposing surfaces creating a closure. The tape tab is preferably removably adhered at a "free end", by the PSA layer, to one surface to allow for adjustment and temporary opening of the closure. The opposing end of the tape tab- is preferably permanently attached to the opposing surface, generally by the same PSA layer. Therefore, a single PSA has the often conflicting objectives of removably or releasably attaching to a first closure surface at a first free end of the tape tab and permanently adhering to a second opposing closure surface at a second opposing end. Complicating these conflicting goals for the single tape tab PSA layer is the fact that the two opposing surfaces are often formed of the same material or of vastly different materials depending on the manufacturer or where the article is produced in the world. This creates significant problems. For example, aggressive adhesives, often necessary to provide permanent attachment to the opposing closure surface at one end of the tape tab, may over aggressively attach the first free end of the tape tab to the first closure surface. Removal of the attached free end can be difficult often causing tearing or distortion of the first closure surface material (typically a thin polyolefin film or web material in a diaper). Conversely, a pressure-sensitive adhesive designed to removably adhere to the first closure surface may not adequately bond to the second opposing closure surface increasing the risk of bond and closure failure.

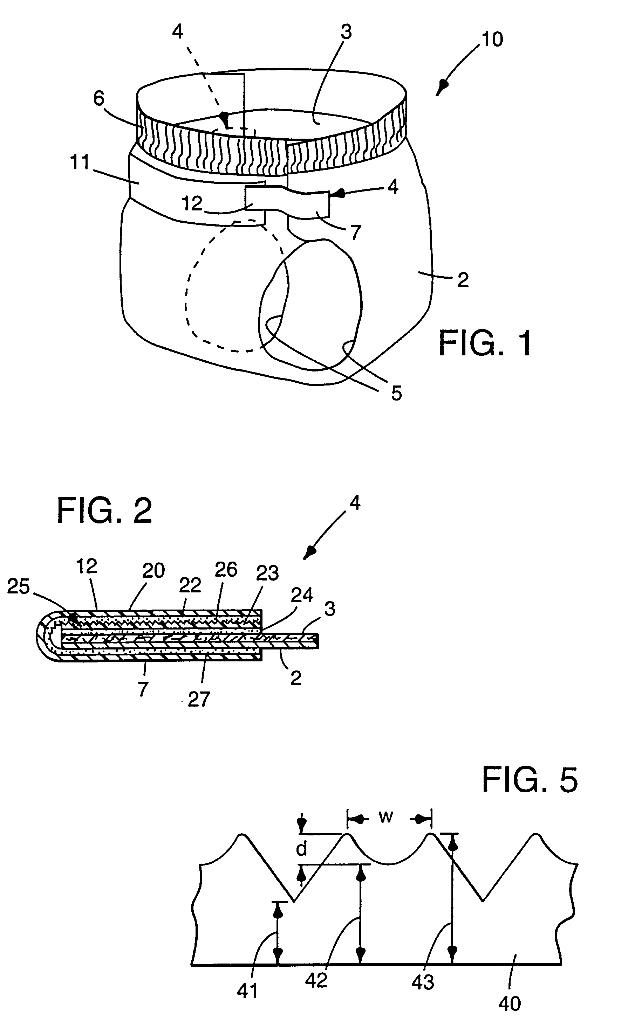

A conventional structure employed in the diaper art to address this problem is shown in Figure 1 where the tape tab 4 is permanently attached at end 7 to a diaper backsheet 2 and removably attached at a free end 12 to a reinforced zone 11 of the backsheet 2, typically a web or film. The free end 12 typically will be placed on a release liner or tape prior to use which release liner or tape is on an inner water-permeable topsheet of the diaper. The reinforcement zone 11 is typically provided by a film or a film-backed pressure-sensitive adhesive tape adhered to the inside or outside face of the liquid impermeable backsheet 2, which reinforcement reduces the tendency of an aggressive adhesive on tape tab 4 free end 12 from tearing or distorting the backsheet 2 when the tape tab free end 12 is removed. This allows a more aggressive adhesive to be used which then allows secure permanent attachment at first end 7. Although this solution is generally quite effective, it is costly and provides additional problems of its own. For example, conventional pressure sensitive adhesive tape tabs, when adhered to the reinforced zone 11, can provide undesirable shocky peels, as described in published PCT Application WO-A- 91/07718. This patent application addresses the problem by providing a specific adhesive formulation on the tape tab which adhesive is designed to securely adhere the tape to the reinforced zone 11 while providing characteristically nonshocky or smooth peel performance. The adhesive described is a particular tackified synthetic rubber, of the A-B block copolymer type, where the elastomeric B block is polyisoprene, containing specific proportions of diblock and, preferably, triblock copolymer.

A further problem with this conventional design is that the reinforcement zone 11 is ineffective if it is inadvertently missed. Larger reinforcement zones could be provided, but are more costly and adversely affect the conformability of the garment. Conventional reinforcement methods are also ineffective with some backsheet materials. Alternative or complimentary solutions to the problems of providing a resealable closure system would be desirable.

An additional general problem with closure systems such as the type described with reference to Figure 1, as well as systems without a reinforcement zone 11, is contamination of the adhesion surfaces by powder, such as talc, or oil, such as baby oil.

DE-A-42 02 704 discloses a diaper comprising a pressure-sensitive adhesive closure system. The closure system comprises a closure tape including a web-like carrier having a pressure-sensitive adhesive layer portion on a first face of its free end, the pressure-sensitive adhesive being in the form of discrete segments and having such an adhesive strength that the peel strength between the tape and the outer film of the diaper is at least 2.5 N/30 mm and the tape can be repeatedly positioned on the outer film of the diaper without damaging said outer film. This prior ast defines the preamble of sidependent claim 1.

EP-A-0 316 601 relates to a pressure-sensitive adhesive closure for a disposable diaper that can exhibit high peel resistance both at low and high speeds while its peelability at high speeds is not excessively shocky. The closure employs a pressure-sensitive adhesive fastening tape, the adhesive of which comprises a blend of an AB block copolymer and tackifying resin. The fastening area of the diaper is a polyolefin layer whose fastening surface has a large number of closely spaced asperities that are at least two micrometers in height and have a jagged appearance.

The present invention is directed toward providing alternative solutions to one or more of the above problems noted in prior art closure systems. The invention is also directed toward a novel closure system which is advantageous in terms of adhesion characteristics such as peel strength while providing an adhesive tape tab which can be used to both permanently and simultaneously refastenably adhere to different surfaces and materials with different adhesion characteristics.

The closure system of the invention is defined in the claims.

It has been found that an advantageous closure system can be provided using a pressure-sensitive adhesive layer, on at least the free end of a fastening tape tab, which adhesive is preferably continuously coated, where the pressure-sensitive adhesive is provided with a deep macrostructured or microstructured outer surface. The adhesive structure comprises peak and valley irregularities where the peak-to-valley average depth is about 10 to 500 micrometers and the peaks are spaced (peak edge-to-peak edge) apart on average less then 500 micrometers . The valley portions are preferably in the form of caldera-like depressions, surrounded by peak structures or a continuous ridge, where the depressions have a depth of at least 5 micrometers from the deepest point of the depression to the average surrounding ridge or peak height.

A process for preparing a deep structure in a pressure-sensitive adhesive layer of a fastening tape tab includes providing a continuous layer of pressure-sensitive adhesive on a tape backing and embossing the structure at least at the free end of the tape by pressing the pressure-sensitive adhesive layer into a structured release tape or film, optionally at elevated temperatures. The release tape or film is provided with a negative of the structure to be imparted to the fastening tape tab free end pressure-sensitive adhesive layer. The free end pressure-sensitive adhesive layer remains in contact with the release tape or film until used in the refastenable adhesive closure system, thereby maintaining or enhancing the fidelity of the adhesive structure until it is used as a refastenable structured adhesive.

A further process for preparing a deep structure in a pressure-sensitive adhesive layer of a tape tab includes providing a continuous layer of pressure-sensitive adhesive on at least the free end of a tape tab backing and embossing the structure at least at the free end of the tape tab by pressing the pressure-sensitive adhesive layer into a structured release tape or film having no release coating or treatment, optionally at elevated temperatures. The release tape or film is provided with structured peak elements having an average peak-to-valley depth greater than the adhesive layer thickness, such that the peak elements alone contact the adhesive and impart structure to the tape tab free end pressure-sensitive adhesive layer. The free end pressure-sensitive adhesive layer remains in contact with the release tape or film peak elements until used in the refastenable adhesive closure system, maintaining or enhancing the fidelity of the adhesive structure until it is used as a refastenable adhesive. The peak elements to adhesive contact area is such that the free end removes from the release tape at a T-peel force of less than 250 N/m, preferably less than 120 N/m, most preferably less than 80 N/m, yet still providing an adequately structured adhesive.

Adhesive structure on a fastening tape tab free end can also be provided prior to attachment to a smooth release film or release tape surface. With this closure system, the pressure-sensitive adhesive structure is either discontinuous or continuous and advantageously provided by caldera-type depressions, which have advantageously been found to be resistant to cold flow over time, or deep structures (e.g., with peak-to-valley heights of from 15 to 500 microns), where the peak height above a minimum unstable peak width or diameter is generally less than the peak to peak average spacing and where the adhesives can also be advantageously resistant to cold flow over time. Other adhesive structures can also be maintained by using an adhesive extremely resistant to cold flow (e.g., highly crosslinked adhesives), although this is less preferred due to loss of pressure-sensitive adhesive properties with highly crosslinked adhesives.

A further aspect of the invention is a smooth peeling tape closure provided by peeling the invention structured adhesive, or an unstructured adhesive, from a surface with peak and valley structures in the peel direction. The peak structures are less than 35 microns with the adhesive and structured surface in intermittent contact with an average of from 20 to 80 interruptions per cm, preferably 30 to 70 interruptions per cm.

The invention is now described in detail in connection with the drawings.
Fig. 1 is a perspective view of a conventional diaper design in which the invention tape tabs find utility.
Fig. 2 is a cross-sectional view of the diaper tape tabs of Fig. 1 with a preferred structured release tape.
Fig. 3 is a top view (150x) of a structured silicone liner for forming the structured adhesive tape tabs of the invention.
Fig. 4-is a top view (200x) of a structured adhesive formed from the liner of Fig. 3.
Fig. 5 is a fragmented schematic side view of the adhesive structure of Fig. 4.
Fig. 6 is a top view (200x) of a structured adhesive formed by exact replication of the liner of Fig. 3.
Fig. 7 is a top view (200x) of a structured adhesive of Fig 4 after stored in roll form, which shows little loss of adhesive texture.

The pressure-sensitive adhesive tape tabs of the inventicn comprise a tape tab substrate provided with a pressure-sensitive adhesive layer having a first pressure-sensitive adhesive region adjacent to one end of the tape tab substrate and a second pressure-sensitive adhesive region adjacent to a second end of the tape substrate. At least the first end adhesive region is a structured pressure-sensitive adhesive layer with peak and valley irregularities or structures or a patterned adhesive. The terms peak or peak structure indicate an adhesive structure or a release tape or film structure having a base and a top portion, the peak top portion being a substantially flat plateau, a ridge or a point.

In a first embodiment, a continuous adhesive layer is provided with adhesive peak structures having an average peak-to-valley depth of generally at least 10 to 500 micrometers, preferably at least 25 micrometers to about 250 micrometers. At peak-to-valley depths below 10 micrometers, typical high-tack pressure-sensitive adhesives used on fastening tape tabs will not adequately retain the structured surface when applied to a mismatched adhesion surface for an extended period of time, undergoing cold flow such as to substantially completely wet the adhesion surface. Textures above 500 micrometers are difficult to produce and provide no added benefits to the adhesion characteristics of the structured adhesive region.

In a second preferred embodiment, the pressure-sensitive adhesive tape first adhesive region is provided with peak and valley irregularities, where the valleys are caldera-like depressions which are substantially completely surrounded by peaks or a continuous ridge structure. The depth between the average surrounding peak or ridge height and the maximum caldera-like depression depth is at least 5 micrometers, preferably at least 10 micrometers. The average caldera width at the average height of the surrounding peak or ridge structures ranges from 5 to 1000 micrometers, however, greater or lesser widths are possible depending on the rheological properties of the particular pressure-sensitive adhesive layer and the average ridge or peak height. With more rigid adhesives, narrower average caldera widths are possible. Also wider average caldera widths are possible for higher average caldera peak or ridge heights. Adhesive structures having these caldera-type depressions are particularly resistant to cold flow and loss of adhesive structure or texture resolution, even with softer pressure-sensitive adhesives, longer contact times with mismatched surfaces or at moderately higher pressures and temperatures.

In a third preferred embodiment, the structured adhesive layer has an open peak and valley texture such as hemispheres, square prisms, rectangular prisms, pyramids, diamonds, tetrahedrons, ellipses, longitudinal ridges forming "V" grooves and cube corners, which can be positive or negative features. By open textures, it is meant that the peaks do not form ridges, plateaus, or the like, surrounding a central enclosed caldera-like depression or valley. The peak-to-valley height or feature height is at least 10 micrometers, preferably at least 15 micrometers, and most preferably at least 25 micrometers. The maximum height is about 250 to 500 micrometers or less depending on the adhesive and the height of the adhesive peak structure above a set width aspect ratio (WAR) and the feature aspect ratio and spacing defined below.

A further aspect of the present invention relates to a process for preparing a deep structure in a pressure-sensitive adhesive layer of a fastening tape tab, which includes providing a continuous layer of pressure-sensitive adhesive on a tape backing and embossing the structure at least at the free end of the tape tab by pressing the pressure-sensitive adhesive layer into a structured release tape backing or film, optionally at elevated temperatures. The release tape backing or film is provided with a negative or mirror image of the peak and valley structure to be imparted to the fastening tape tab free end pressure-sensitive adhesive layer. The free end pressure-sensitive adhesive layer remains in contact with the structured release tape backing or film until used in the refastenable adhesive closure system, thereby maintaining or enhancing the fidelity of the adhesive structure until it is used as a refastenable adhesive.

The width aspect ratio (WAR) is the ratio of the mean width or diameter (Dₓ) of a feature at a given height (X) of a feature to the feature height above that given height(H-X; H is total feature height).

For a triangular feature (e.g., a cone, pyramid or V-groove), the WAR will be a constant for a given triangle at any given height (X). The WAR is indicative of the stability of a given structure and its slope. A WAR below about 0.2 is considered to be unstable (H-Xᵤ, Xᵤ = unstable WAR height) for the preferred rubber-resin adhesives of the invention. Unstable features are those which will tend to move laterally or fall into the adhesive mass. Preferably, the WAR is above 1.

The minimum spacing of the features or structures is dependent on the height of the feature above an unstable WAR. Preferably the next highest peak will be greater than 0.2 times the height above the unstable WAR height(H-Xᵤ) of the peak, preferably at least 0.5 times the unstable WAR height.

The spacing of the peaks also depends on the conformability of the fastening tape, the substrate being adhered to and the peak height. With low peak heights and/or more conformable tapes or more conformable adhered to substrates, the peak top portions should be spaced closer to prevent excessive adhesion of adhesive in the valley portions, between the peaks, to the adhered to substrates. The top portions create adhesive contact regions. Generally, the best results are obtained when the top portions of the peaks are less than 500 microns apart, peak top portion-to-peak top portion.

Adhesives particularly well suited for use in forming the structured pressure-sensitive adhesive layer for a fastening tab, particularly a diaper fastening tab, included tackified elastomers where the elastomer is an A-B-type block copolymer, wherein the A block(s) and B block(s) are configured in linear, radial, or star configurations. The A block is a monoalkenyl arene, preferably polystyrene, having a molecular weight distribution between 4,000 and 50,000, preferably between 7,000 and 30,000. The A block content is preferably about 10-50 percent, preferably 10-30 percent. Other suitable A blocks may be formed from alphamethylstyrene, t-butyl styrene and other ring alkylated styrenes, as well as mixtures thereof. The B block is an elastomeric conjugated diene, having an average molecular weight from about 5,000 to 500,000, preferably from 50,000 to about 200,000, which B blocks can be further hydrogenated or functionalized. The elastomer preferably comprises at least 15 weight percent, more preferably at least 25 weight percent, of either block copolymers having B end blocks, such as A-B diblock copolymers, or pure B elastomers, most preferred are A-B-type block copolymers having B end blocks.

The tackifying component for the elastomer-based adhesive generally comprises solid tackifying resin used alone or in combination with a liquid tackifying resin and/or liquid plasticizer. Preferably, the tackifying resin is selected from the group of resins at least partially compatible with the diene B block portion of the elastomeric block copolymer. Such tackifying resins include those aliphatic hydrocarbons made from polymerization of a feed stream consisting mainly of unsaturated species containing 4 to 6 carbon atoms; rosin esters or rosin acids; mixed aliphatic/aromatic tackifying resins; polyterpene tackifiers; and hydrogenated tackifying resins. The hydrogenated resins may include resins made from the polymerization and subsequent hydrogenation of a feedstock consisting mainly of dicyclopentadiene; resins produced from the polymerization and subsequent hydrogenation of pure aromatic feed stock such as styrene; resins produced from the polymerization and subsequent hydrogenation of an unsaturated aromatic feed stream, wherein the feed stream consists mainly of species containing 7 to 10 carbon atoms; hydrogenated polyterpene resins; and hydrogenated aliphatic and/or aliphatic aromatic resins. Preferred tackifying resins include the aliphatic hydrocarbon resins and the hydrogenated resins. A relatively minor portion of the tackifying resin can include resins compatible with the A block, generally termed end block reinforcing resins. Generally, these end block resins are formed from aromatic species. Suitable liquid plasticizers include naphthenic oils, paraffinic oils, aromatic oils and mineral oils. These adhesives have the requisite adhesive properties for use in a fastening tab and are well suited to forming structured adhesive surfaces by the preferred processes of the invention. Where the structured adhesive layer peak elements are of a shape less resistant to shape deformation, and/or where the adhesive is used under conditions where the adhesive will be exposed to a non-mated surface for an extended period of time and/or where the adhesive is under significant pressure and/or temperature, it may be desirable to reinforce the adhesive to make it more resistant to cold flow. Reinforcement need only be to a degree sufficient for the adhesive to retain the structure up to the maximum estimated time before use. This reinforcement can be by suitable use of mechanically reinforcing fillers or crosslinking the adhesive such as by thermal or UV radiation, optionally employing suitable crosslinking agents. Generally, however, crosslinking is undesirable as it tends to reduce the level of tack to a point where the adhesive is not well suited for use as a pressure-sensitive adhesive. Physical reinforcement is also possible such as by mating the adhesive to a mirror image structured liner or release surface until ready for temporary use or by using a structured tape tab backing. A structured tape tab backing would have a shape matching that of the overlying structured adhesive.

Other suitable adhesives include acrylate pressure-sensitive adhesives, which can be modified to increase resistance to cold flow, such as described in U.S. Patent No. 4,554,324 which grafts onto a conventional acrylate chain a high Tg polymeric block capable of physically crosslinking the acrylate chains. These high Tg blocks can be the A blocks described above. These high Tg blocks have a molecular weight of from 2,000 to 30,000. Other suitable tackified elastomeric adhesives are also suitable for certain applications.

The adhesive fastening tape tab backing substrate can be any conventional tape backing or flexible material including thermoplastic films, woven materials, consolidated nonwoven webs, or the like.

Figure 2 depicts a preferred method for forming the invention structured adhesive layer on a diaper fastening tape tab free end for use on a diaper of conventional design, such as the one depicted in Figure 1. The fastening tape tab 4 backing 20 is coated with a pressure-sensitive adhesive layer. The backing 20 is then permanently attached at end 7 to, e.g., a diaper backsheet 2, typically a thin water-impermeable polyethylene or polyolefin film, with a pressure-sensitive adhesive layer region 27. The free end 12 of the pressure-sensitive adhesive fastening tape tab 4 is pressed into a release tape or film 25 prior to attachment to the diaper frontal region 11. The release tape or film 25 would be permanently attached to the, e.g., inner facing 3 of the diaper, preferably through a pressure-sensitive adhesive layer 24. The inner facing 3 would eventually be a liquid-permeable material, such as a nonwoven web. The inner facing 3 and the backsheet 2 taken together would comprise the second closure surface. The release tape or film 25 backing 23 is provided with a structured surface 26, which is a negative of the surface peak-and-valley structure to be imparted to the pressure-sensitive adhesive layer region 22 at the free end 12 of diaper fastening tape tab 4. The adhesive layer region 22 is pressed into the release tape backing structure during the manufacture of the diaper under suitable pressure, such as by a pressure roller, which may be heated. The pressure generally would be greater than about 100 Pascals, preferably at least 400 KPascals. This method of manufacture is particularly desirable where the adhesive texture is other than that of a caldera-like depression or having a peak feature height at the lower end of the described ranges, as the adhesive structure fidelity is maintained until immediately prior to use. Therefore, the adhesive need only retain its structure for the short period of time that is used to effect closure, e.g., of a diaper, typically less than 24 hours and generally less than about 8 hours.

In a preferred embodiment the structured release film or tape 25 has an average structure height(peak to valley) greater than the adhesive thickness and having peak structures. The total peak structure top portion area and average peak-to-peak spacing are such that the adhesive contacts only a small portion of the release tape backing through the peak top portions providing a T-peel value of less than about 250 N/m, preferably less than 120 N/m, most preferably less than 80 N/m. The release tape 25 in this embodiment can act as a release tape without any release coating. Typically where no release coating is used the adhesive would contact less than 25 percent of the release tape or liner backing surface, across a planar cross section of the backing face in the peak top portions contact with the adhesive, preferably less than 20 percent. A preferred structure for the release liner would be tapered peaks or ridges where the peaks or ridges taper continuously from the top portion of the peak or ridge to the peak or ridge base portions, continuous taper meaning that the peaks or ridges have no substantial head, or hook or like structures such as found on a male mechanical fastening structure. A preferred peak structure would be upstanding stems or conical peaks.

Alternative methods for forming adhesive structures more resistant to deformation are employed where the release tape or film has a backing with a smooth surface or a surface structure that does not match the adhesive structure (not shown). Preferred alternatives for this diaper tape tab closure system are to form the caldera-like depression adhesive structures or have adhesive structure peak-to-valley heights of greater than 25 microns. Prestructured adhesives could also be made flow resistant, such as by crosslinking and made into a variety of shapes. However, generally, crosslinked adhesives are not preferred for use in diaper applications due to their low tack and inability to adequately adhere in a closure system without the risk of closure failure by adhesive tab pop-off or shear failure.

Prestructured or preformed (i.e., prior to contact with a mismatched surface were the adhesive is adhered for an extended period of time prior to use) adhesives can be produced by embossing a smooth adhesive layer with a suitably structured release liner that is subsequently removed. Alternatively, the structured pressure-sensitive adhesive can be produced by coating or casting an adhesive onto a structured release liner, such as by hot-melt or solution coating or casting. A backing film would then be brought into contact with the adhesive layer coated structured release liner. This backing layer is then removed, with the adhesive layer, from the structured release liner (which liner would have a surface coated or selected to have a lower degree of adhesion to the adhesive than the backing).

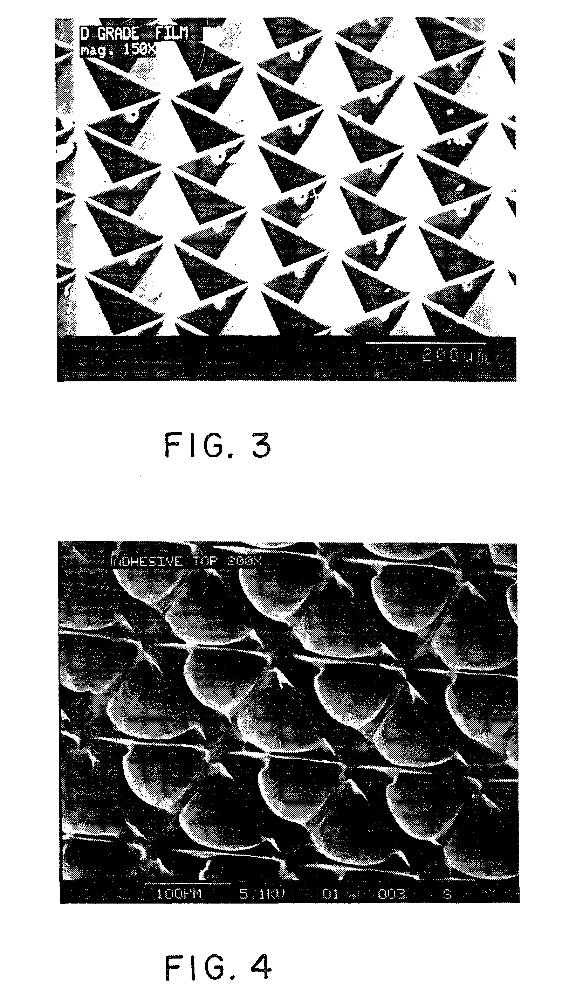

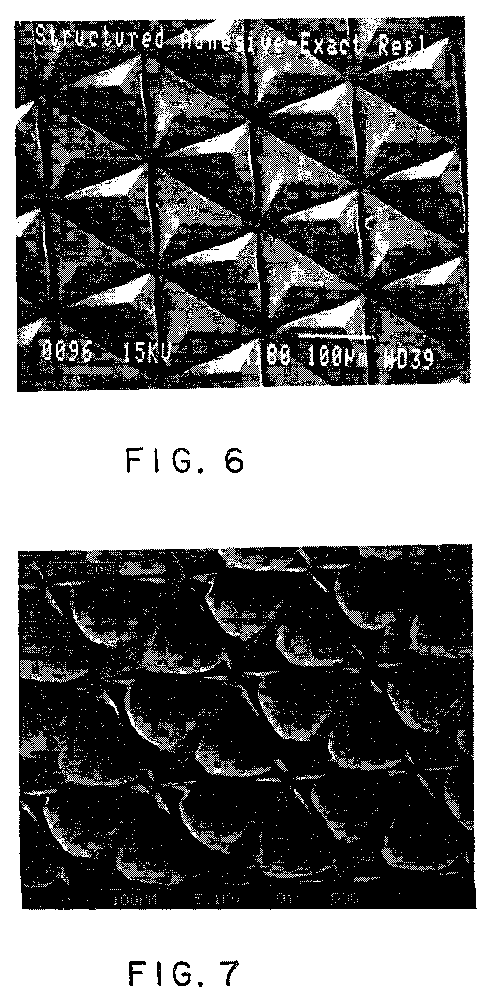

A method for forming the caldera-like depressions has been found when using a structured silicone liner, such as the cube corner design of Figure 3. The liner is solvent coated with an elastomeric-based pressure-sensitive adhesive and allowed to dry prior to lamination, depending on the adhesive viscosity the resulting adhesive can form into structures with collapsed peaks creating caldera-like structures, such as shown by the scanning electron micrograph of Figure 4 and schematically shown in the partial fragmentary cross-section of Figure 5. Alternatively, the adhesive can be coated in a manner to exactly replicate the structured liner of Figure 3 as shown in Figure 6. In Figure 5 the adhesive depths 41, 42 and 43 at various points provide a peak-and-valley structure with caldera-like depressions having a defined valley or caldera width-to-depth ratio (WND), where the width is the average height of the depression at the average width of the surrounding ridges or peaks. While not wishing to be bound by theory, it is believed that the collapsed peaks result due to incomplete wetting of the adhesive in the depressions of the liner trapping microbubbles, and possibly solvent drying effects. A more exact replication of a particular microstructured liner can be obtained where the pressure-sensitive adhesive is formed by a lower viscosity adhesive solution or a reactive solution, which forms into a pressure-sensitive adhesive on the microstructured liner, such as by use of a photoinitiator, as described in U.S. Patent No. 4,181,752.

The structured adhesive layer 22 of the invention would be used by adhering to a second opposing closure member surface for releasable or resealable attachment, which in a diaper would be a frontal region 11, as shown in Figure 1, which frontal region 11 could be provided with reinforcement. In a particular preferred arrangement, a reinforced frontal region 11 would be provided by a smooth or matte reinforcement film applied, with a hot melt or pressure-sensitive adhesive (where the film is a tape backing), to the outside of the diaper water impermeable backsheet 2 layer. When the structured adhesive 22 is adhered to this reinforcement film surface, it generally provides a smooth or nonshocky resealable peel (shocky is generally understood in the art as a peel which is jerky and creates significant audible noise) with generally high peel values compared to a similar smooth adhesive surface of the same pressure-sensitive adhesive. The opposing end 7 would then preferably be provided with a smooth or less structured adhesive surface in pressure sensitive adhesive region 27. This allows for secure permanent attachment to the diaper backsheet 2. In this manner the same adhesive can be used to securely adhere one end of a tape tab to one surface and through surface modification releasable adhere the tape tab free end to a second surface. However different adhesives can be used in adhesive regions 22 and 27. Preferred for diaper tapes are the synthetic rubber-based adhesives using an A-B-type block copolymer as described above. The adhesive structure may also provide some measure of contamination tolerance against oil and talc, or powder, to the adhesive fastening tab.

The percentage of surface area structured at free end 12 of the fastening tab 4 generally is close to 100 percent. However, the peak top portion or land contact area of the structured adhesive (e.g., the peak area of the projecting peak structures, or land areas for concave or depression structures) is generally less than 75 percent preferably less than 50 percent of the free end adhesive region planar cross section(across the top portions of the adhesive peaks).

Certain of the advantages of the invention can be obtained by providing the structured adhesive at only a portion of free end 12. For example,providing the structured adhesive only at the leading or outer edge provides improved initial smooth peel performance while providing a portion of the adhesive layer with a smooth surface so as to provide adhesion advantages associated with this particular surface, such as high shear performance.

Generally, the width of the outward projecting. adhesive peak structures will be narrower at the peak top portion than at the peak base, and will typically taper continuously from peak top portion to base, generally at an angle of about 10 to 60 degrees, preferably 25 to 50 degrees. A general WAR of the overall base width to peak height would be from about 0.4-10, most preferably from about 1.0-5.

With a caldera-like depression, the width-to-depth (WND) (the width being the average width of the depression at the average height of the surrounding ridges or peaks) ratio for the depression typically will be from about 1 to 100, preferably 5 to 75 with a minimum depth of at least 5 microns. For the open peak and valley structure irregularities the average width(i.e. the average distance between the edges of the top portions)-to-depth(i.e. the average valley depth) ratio is from 1 to 50, preferable from 5 to 30, with a minimum average depth of 10 microns.

The average spacing distance between neighboring upstanding adhesive peak structures(also corresponding to the average valley or depression width) depends on the adhesive employed and the desired level of adhesion performance required for the particular combination of materials used for the closure system adhesion surfaces. The spacing and distance between various adhesive peak structures can also be selected so as to match structures on the adhesion surface to which the adhesive is applied (e.g., surface 11 in the diaper construction of Figure 1) to provide increased adhesion performance.

The nearest neighbor distance between features can also be specified with a spacing aspect ratio (SAR) given by the ratio of center-to-center nearest neighbor distance to feature width. The minimum value of the SAR is one corresponding to side-to-side feature touching. Generally, the SAR would be less than about 1:100, preferably 1:50, most preferably 1:20. If the SAR is too great, the adhesive features will tend to flatten and lose their inner-spacial gaps.

The structure on a release tape or liner can be formed by casting a pattern onto a thermoplastic film or casting a thermoset or crosslinkable material on a positive mold formed by engraving, etching, sandblasting or the use of an inherently irregular surfaced material, such as a woven or nonwoven web, or the like. A release tape or liner can be formed by casting or embossing of a thermoplastic film, which can be followed by coating with a suitable release agent coating if required for the release liner to cleanly release from the adhesive. With a post embossing release layer coating, the cast or embossed pattern should be somewhat exaggerated as the release coating will tend to eliminate sharp corners and partially fill the liner valleys. In a preferred embodiment the thermoplastic film has a previously applied thermoplastic release coating or layer prior to forming the microstructure in the film by casting or embossing the film with a positive mold. These thermoplastic release layers are provided by thermoplastic polymers such as polyolefins grafted on with silicone or fluorochemical-containing side groups or blended with release agents.

A suitable release layer composition is described in U.S. Patent No. 5,169,900 where dimethylpolysiloxane (PDMS) terminated with a silanol is grafted onto polyethylene. The films exemplified in this patent could function as structured release liners or tapes or structured liners provided that they are embossed or cast prior to the silicone curing. A coextruded polyolefin grafted with reactive silicone is described in EP -A- 484, 093 where the grafted material is coextruded with a polyolefin. A blend layer of PDMS with polyolefins is described in U.S. Patent No. 4,673,611, which could also serve as an embossable release liner backing.

A suitable fluorochemical grafted polyolefin film is described in WO-A-92/15626, which films could also function as release liners or microstructured liners. In this document, a fluoroaliphatic-containing moiety, e.g., N-butyl perfluorooctanesulfonamido-ethyl acrylate (BuFOSEA), is grafted onto a polyolefin in the melt phase of an extruder and the presence of a free radical initiator(s).

The following examples are currently contemplated preferred nodes for carrying out the invention and should not be considered as limiting thereof unless otherwise indicated.

The following tests were used to evaluate the microstructured adhesive tapes.

### 135 Degree Peel Adhesion

This test is a modified version of PSTC-5. The test was carried out at constant temperature and humidity (21° and 50 percent relative humidity) using a constant rate Instron™ tensile tester. The film sample to be tested was securely adhered to a 2 inch x 5 inch (5.1 cm x 12.7 cm) steel panel using a double-coated adhesive tape. Within 60 minutes after securing the film sample to the steel panel a 1 inch (2.54 cm) wide strip of test tape was then placed adhesive side down onto the film substrate and was rolled down onto the film substrate using two passes of a 4.5 1b (2000 gm) hard rubber roller. The peel rate was 12 inches (30.5 cm) per minute. The force (N/m) required to remove the fastening tape from the test substrate is reported in the Tables. Reported values are averages of at least two tests.

### EXAMPLES

### Example 1

A pressure-sensitive adhesive was solution coated onto a silicone rubber liner having a surface microstructure (cubed corner) as shown in the scanning electron micrograph (SEM) photo in Fig. 3, the feature depth is about 60 microns The adhesive composition was 45.5 weight percent Kraton™ 1111 (a polystyrene-polyisoprene linear block copolymer available from Shell Chemical Co. having approximately 15 percent diblock and 85 percent triblock, and a styrene content of about 21 percent), 46.7 weight percent Wingtack™ Plus (a solid C₅ tackifying resin available from Goodyear Chemical Co.), 6.3 weight percent Shellflex™ Oil (a naphthenic oil available from Shell Chemical Co.), and 0.75 weight percent each of Irganox™ 1010 and 1076 (antioxidants available from Ciba-Geigy). The adhesive was coated from a 48 percent solids solution of a 1:3 mixture of heptane and toluene. The coated film was then dried in a 150°F (65°C) oven for ten minutes. The adhesive coated film was then laminated to a 4 mil (100 microns) film of an ethylene-propylene impact copolymer (resin 7C-50 available from Shell Chemical Co.). After lamination the silicone liner was removed from the adhesive. The thickness of the microstructured adhesive layer on the cast film was 37.5 microns. An exact replication of the microstructured surface of the silicone liner was not achieved. A SEM photo of the resultant microstructured adhesive pattern is shown in Fig. 4 indicates that the peak to valley distance is about 30 micron. The pattern of the microstructured adhesive is best described as being that of tetrahedra with a concave caldera-like depression. It is believed that this is likely due to solvent evaporation and adhesive surface wetting effects. A sample of the microstructured adhesive tape was then adhered to a glass slide and pressure was applied by rubbing back and forth with an index finger. After peeling the tape away from the glass slide the adhesive surface was examined by optical microscopy and the surface structure had remained substantially intact. This demonstrates that the microstructure on the adhesive does not easily lose it's fidelity due to cold flow.

### Example 2 and Comparative Example 3

Additional tape samples were produced by the method described in Example 1 and were tested for 135 degree peel adhesion from various substrates: 1) a smooth tape surface of biaxially oriented polypropylene (BOPP) coated with a urethane low adhesion backsize (LAB) release coating, 2) a matte finished cast film of an ethylene-polypropylene impact copolymer(cast PE-PP) and 3) the raised surface of an embossed non-LAB coated polypropylene film having a pattern of convex hemispheres (100 lines per inch), the hemispheres being about 30 microns high.

For comparison, tape samples having smooth adhesive surfaces were also tested. The smooth tape samples were prepared by solvent coating a solution of the adhesive composition described in Example 1 to the smoother side of the cast matte/matte film described in Example 1. The adhesive thickness of the smooth adhesive tape samples was 38.3 microns. 135 degree peel adhesion results are given in Table I for both the replicated adhesive tapes (Example 2) and smooth adhesive tapes (Comparative Example 3).

**TABLE I**

| Example | Structured Surface | Smooth Surface | BOPP | Cast PE-PP | 100 lpi PP |
|---|---|---|---|---|---|
| 2 | x | | 118 | 259 | 233 |
| C3 | | x | 69¹ | 508 | 431 |

| | | | | | |
|---|---|---|---|---|---|
| ¹ shocky peel | | | | | |

The results indicate that higher and smoother peels can be obtained with the microstructured adhesive against BOPP compared to the smooth adhesive which gave a lower peel adhesion value and was shocky. Lower peel values were obtained for the microstructured adhesive compared to the smooth adhesive for the cast ethylene-propylene film and the 100 lpi embossed polypropylene film but the peel values were still very adequate and were non-shocky. The lower peels against the structured film was believed to be due to mismatching of the adhesive and film structure lowering the adhesive contact area which also indicates that the adhesive microstructure is maintained on these surfaces.

SEM photomicrographs were also taken of the microstructured adhesive surface of the Example 2 tapes after peeling the tapes from the substrates (BOPP, Cast PE-PP, & 100 lpi PP). The photomicrographs showed that all samples retained their surface microstructure. This demonstrates resistance to adhesive cold flow for this concave caldera-type depression microstructure.

### Example 4 and Comparative Example 5

Samples of microstructured adhesive tapes were prepared as described in Example 1 except that the coating thickness of the adhesive was 25 microns. SEM photomicrographs were taken of the adhesive surface. The tape was then laid adhesive side down onto the urethane release coated side of a release tape which had a surface roughness of 111 Ra. This composite was then run through a set of lamination rolls set at 60 psi. The release tape was then removed from the laminate and the structure of the adhesive surface was examined by SEM to determine if any change had occurred in the adhesive surface structure due to adhesive cold flow.

For comparison, an adhesive tape having a relatively smooth adhesive surface was subjected to the same treatment and was also examined by SEM. The adhesive composition of this Comparative Example 5 (C5) tape was identical to that of the adhesive on the Example 4 microstructured adhesive tape. The Comparative Example 5 tape backing was a clear matte/matte cast film of an ethylene-propylene impact copolymer on the adhesive coated face. The adhesive coating thickness was 25.8 microns.

The SEM photomicrographs before and after lamination indicated that the smooth adhesive surface of the C5 tape, which was relatively smooth prior to lamination, had become matted after lamination, picking up some of the structural features of the 111 Ra release film. In contrast, the microstructured adhesive tape of (Example 4), having an adhesive surface pattern of concave depressions maintained its original replication fidelity after lamination.

These results indicate that adhesive cold flow can be influenced simply by changing the surface structure of the adhesive rather than by changing the adhesive composition.

### Example 6

A 2 inch x 50 yard (5.1 cm x 45.7 m) length of tape was prepared in a manner similar to the sample in Example 1 except that, after drying, the 4 mil cast film/adhesive/silicone liner laminate was wound into a roll. The liner was then later removed when the roll of tape was being slit into narrower widths and the tape was wound into a roll such that the microstructured adhesive face was in contact with the cast film.

SEM photomicrographs were taken initially and after two months aging at 75°F (24°C) in a roll without the silicone liner. The photomicrograph showed that almost no loss in the microstructured pattern on the adhesive surface had occurred after aging in a roll (Fig. 7). This further demonstrates that microstructured adhesive has remarkable resistance to cold flow when the adhesive has a surface microstructure of concave depressions.

### Examples 7 and 8

Microstructured adhesive tape samples were prepared as described in Example 1 except that the silicone rubber liner had a V-groove microstructure with a peak to valley height of about 50 microns. The V groove microstructure was replicated almost exactly giving an adhesive with a peak to valley height of about 50 microns.

The tape samples were tested for 135 degree peel adhesion in both the machine and cross directions (MD and CD)against BOPP film and cast PE-PP film. The results are given in Table II. All peels were non-shocky.

**TABLE II**

| Example | BOPP | Cast PE-PP | 135 Deg Peel (MD) | 135 Deg Peel (CD) |
|---|---|---|---|---|
| 7 | x | | 75 | 55 |
| 8 | | x | 360 | 349 |

Comparison of these results to those obtained for the concave tetrahedra microstructured adhesive (Tables I and II) illustrate that for a given adhesive composition the adhesive properties of the tape can be altered by changing the surface structure pattern of the adhesive. However the structured adhesive promotes smooth peel performance against a variety of substrates at a wide range of peel values.

### Example 9

Adhesive tape samples having a V-groove microstructured adhesive surface were prepared as described in Example 7. The tape samples were tested for adhesive cold flow as described in Example 3.

SEM photomicrographs showed that the V-groove microstructured adhesive surface was unchanged after lamination indicating initial resistance to adhesive cold flow.

### Example 10 and Comparative Example 11

Microstructured adhesive tape samples were prepared as described in Example 1 except that the adhesive composition varied slightly and the adhesive was coated from a lower percent solids solution. The adhesive composition was 42 weight percent Kraton™ 1111, 45 weight percent Wingtack™ Plus, 13 weight percent Shellflex™ 371, and less than 1 weight percent of Irganox™ 1076 antioxidant. The adhesive was coated from a 33 percent solids solution in toluene. This resulted in an exact replication of the cubed corner microstructure as shown by the SEM photomicrograph of the adhesive surface in Fig. 6. The coating thickness of the adhesive was approximately 25 microns, however the peak height of the structured adhesive was about 60 microns.

For comparison, tape samples having smooth adhesive surfaces were also prepared from the 33 percent solids adhesive solution. The coating thickness of the adhesive for these tape samples was approximately 33 microns. The microstructured adhesive and the smooth adhesive tape samples were tested for 135 degree peel adhesion from a smooth BOPP film having a urethane LAB release coating.

**TABLE III**

| Example | Structured surface | Smooth surface | BOPP |
|---|---|---|---|
| 10 | x | | 78 |
| 11 | | x | 121¹ |

| | | | |
|---|---|---|---|
| ¹ shocky peel | | | |

Smooth peels were obtained with the adhesive tapes having the cubed corner microstructured surface compared to the adhesive tapes having the smooth adhesive surface which resulted in shocky peels.

This example demonstrates that lowering the adhesive viscosity (by decreasing the percent solids of the adhesive solution) enhances the wetting efficiency of the adhesive, resulting in a more perfect replication of the microstructure.

### Examples 12 and 13

Smooth adhesive tape samples were prepared as described in Comparative Example 11 above except that the adhesive was coated from a 50 percent solids solution in toluene. The adhesive coating thickness was 37.5 microns. The smooth adhesive tapes were then placed adhesive side down onto a 50 micron thick embossed polypropylene film or liner having a pattern of about 30-micron high hemispherical depressions (65 lines per inch) which had been coated with a urethane release agent. The release agent used in this and the other examples was an organopolysiloxane polyurea block copolymer (such as described in U.S. patent No. 5,214,119) which was coated onto the film as a 5 percent solution. The laminates were then rolled down two times with a 4.5 1b (2000 gm) hard rubber roller. The adhesive tapes were allowed to sit against the embossed film for at least 24 hours at constant temperature and humidity conditions (23°C, 50 percent relative humidity) after which the embossed film was peeled off of the adhesive tapes. Samples were prepared using both sides of the embossed film resulting in tapes having microstructured adhesive patterns of either concave or convex hemispheres of less than about 30 microns. The microstructured adhesive tape samples were then tested for 135 degree peel adhesion from a smooth BOPP film having the urethane LAB release coating. Peels were smooth for both the concave hemispherical pattern and the convex hemispherical pattern.

**TABLE IV**

| Example | Concave hemispheres | Convex hemispheres | BOPP |
|---|---|---|---|
| 12 | x | | 157 |
| 13 | | x | 204 |

These examples demonstrate that a microstructured adhesive surface can be prepared simply by embossing a smooth adhesive surface against a release treated microstructured film or liner. They also demonstrate that tapes having adhesive microstructures of concave or convex hemispheres result in smooth peel characteristics.

### Examples 14 - 20

A backing having a stem or pin-shaped microstructure was prepared by extruding an ethylene-propylene impact copolymer resin (30 MFI, SRD7-463 available from Shell Chemical Co.) into the holes or cavities of a mold, which can be made in accordance with the disclosure in U.S. Patent No. 4,959,265. The physical dimensions of the microstructured backing are given in Table V (dimensions of the mold are given in parentheses). All values are in microns.

**TABLE V**

| Example | Pin height | Pin diameter (at tip) | Pin diameter (at base) | Pin spacing* |
|---|---|---|---|---|
| 14 | 80(90) | 35(75) | 125(160) | 250 |
| 15 | 85(135) | 80(60) | 160(175) | 250 |
| 16 | 120(200) | 50(25) | 160(175) | 250 |
| 17 | 185(325) | 110(40) | 160(175) | 250 |
| 18 | 150(370) | 75(70) | 110(125) | 525 |
| 19 | 80(160) | 60(20) | 130(160) | 800 |
| 20 | 45(60) | 40(60) | 110(145) | 800 |

| | | | | |
|---|---|---|---|---|
| *center-to-center | | | | |

An adhesive tape having a smooth surface and coating thickness of 50 microns was prepared as described for Example 13 above. The adhesive tape was placed adhesive side down against the pins of the microstructured backing. The laminate was then rolled down two times with a 4.5 1b (2000 gm) hard rubber roller. The laminates were allowed to sit for at least 24 hours at constant temperature and humidity conditions (23°C, 50 percent relative humidity) after which the adhesive tapes were peeled off of the pin-shaped microstructured backing. The surface texture of the adhesive was that of cup-shaped depressions. The tape samples were tested for 135 degree peel adhesion from a smooth BOPP film coated with a urethane LAB release coating. The peel adhesion values for examples 14-17 ranged from 206-216 N/m. and all peels were smooth. For examples 18-20 the 135 degree peels were shocky indicating that the adhesive was insufficiently microstructured due to the wide pin spacing (less than about 25 percent of the adhesive containing pin formed structural depressions).

To demonstrate the feasibility of using the pin-shaped microstructured backing as a release tape surface, the laminates prepared above were tested for "T-peel". The "T-peel" test was run in a manner similar to the 135 degree peel adhesion test described above except that the test substrate was the microstructured backing and it was not secured to a steel panel during testing. The peel values ranged from 44-83 N/m for examples 14-16, which would be suitable for use as a release tape. "T-peel" data was also obtained after aging for seven days under a 1600 gm load at room temperature conditions. Peel values changed only slightly, ranging from 54-89 N/m. For examples 17 and 20 the T-peels were 139 and 270 N/m generally high for release liners in most applications. Examples 18 and 19 had the lowest T-peel values, 19 and 10 N/m, respectively.

These examples demonstrate that a film or backing having a microstructured surface can function as a non-LABed release tape or backing for an adhesive tape and also serves to impart a microstructure to the surface of the adhesive.

### Example 21

A hot melt pressure sensitive adhesive (40% Kraton™ 1111, 12% Shellflex™ 371, and 48% Wingtack™ Plus) was coated onto a 4 mil (102 micron) thick polyvinyl alcohol film backing. The adhesive was coated as a series of regularly spaced square prisms, each of which are about 0.02 by 0.02 inches (0.051 by 0.051 cm) and have a peak height of about 0.014 inches(0.036 cm). The center to center spacing or the prisms was about 0.033 inches (0.084 cm). When tested for 135 degree peel, from a LAB coated BOPP film, the pattern coated tape gave a peel force of 161 N/m and was smooth. A nonpattern coated control gave a 135 degree peel of 97 N/m and was shocky.

### Examples 22-23 and Comparative Examples 24-26

Smooth tape samples, 38 microns thick, were prepared as described in Example 2 except that the adhesive composition was 55 weight percent Kraton™ 1119, 41 percent Wingtack™ Plus, 4 weight percent Shellflex™ 371 and less than 1 weight percent of Irganox™ 1076 antioxidant. The adhesive tapes were then tested for 180° peel adhesion D1876-76 except as follows; each pressure-sensitive adhesive tape sample was one inch (2.54 cm) by 8 inches (20.32 cm), and was laminated by its adhesive layer to the face of a test sample of film [2.5 inches (6.35 cm) by 8 inches (20.32 cm)] using two passes of a 4.5-pound (2.04 kg) mechanical roller (at 30 cm/min.) conforming to Federal Standard 147. The whole was then inserted into the jaws of the tensile tester along with a steel panel [2 inches (5.08 cm) by 5 inches (12.7 cm) and 0.0625 inches (0.159 cm) in thickness], against which the film rested in order to keep it aligned with the direction of movement of the jaws. The peel rates were set at 30.5 cm/min (12in/min) and 1015 cm/min (400in/min) against the following film surfaces:
1) a smooth surface of biaxially oriented polypropylene(BOPP) coated with a urethane low adhesion backsize release coating(Comparative Example 23);
2) a microrough cast polypropylene film comprising closely spaced jagged asperities such as exemplified in U.S. Patent No. 4,861,635 (Comparative Example 24);
3) the embossed face, comprising a pattern of slightly raised projections (<10 µms) at more than 200 lines of projections per inch (80 per cm), of a 2.0 mil (51 µm) thick polypropylene film from Edison Plastics (Comparative Example 25);
4) a 2.2 mil (56 µm) thick cast polypropylene (Shell 7CO5N) film with a plurality of raised projections about 30 µm high at about 85 lines of projections per inch (34 per cm) (Example 22);
5) a 2.2 mil (56 µm) thick cast polypropylene (Shell 7CO5N) film with a plurality of raised projections about 25 µm high at about 110 lines of projections per inch (43 per cm) (Example 23).

The peel adhesion results in N/m of the smooth adhesive tape against these surfaces are given in Table VI below:

**TABLE VI**

| Example | Structure height in µm | Percent adhesive contact | 30.5 cm/min | 1015 cm/min |
|---|---|---|---|---|
| comparative 23 | - | ∼100 | 310¹ | 66¹ |
| comparative 24 | - | ∼100+ | 586³ | -⁴ |
| comparative 25 | <10 | -75 | 486² | -⁴ |
| example 22 | 30 | -40 | 369² | 347² |
| example 23 | 25 | ∼45 | 450² | 409² |

| | | | | |
|---|---|---|---|---|
| ¹ shocky | | | | |
| ² smooth | | | | |
| ³ marginal | | | | |
| ⁴ ripped | | | | |

The results indicate that the example surfaces provided smooth, low noise peels in a desired performance range of 150-415 N/m, even at the higher peel rate, for a typical diaper fastening tape adhesive formulation. This smooth peel can be achieved by use topologically microstructured surfaces with a plurality of projections of less than about 35 microns high, which projections contact less than 60 percent of the total surface area of the adhesive on the tape and preferably have a height of at least 10 microns. These projections adequately disrupt the peel front and yield smoother or lower noise peels. The above peel values indicate that if the projection structure is shallow (<10 microns), with a PSA more than 35 µm thick, there is a higher percent of the adhesive surface in contact with the structured surface (as in comparative example 22) which may lead to ripping of a structured film when removing the adhesive tape from the film at high peel speeds. The height of the structures can also be used as a variable for manipulating peel adhesive forces and the percent adhesive surface contact in conjunction with the adhesive thickness.

These examples illustrate another aspect of the present invention where a structured surface acts as a smooth peeling surface for either a structured or nonstructured adhesive on a tape. This smooth peel performance can even be obtained when the adhesive thickness is greater than the height of the structures. This smooth peel performance is most noted against shocky peeling rubber-resin type adhesives such as used in conventional diaper tapes.

The direct adhesive to structured surface contact area should be less than 70 percent of the planar cross-sectional surface area in the adhesive contact area, preferably less than 60 percent and also preferably greater than 20 percent. The structures on the structured surface are preferably in the form of peaks and valleys having on average from about 20 to 80 peak structures per linear centimeter in at least one direction, preferably 30 to 70 peak structures/cm in at least one direction. The peak structure can be in any form including mounds, cones, mushrooms, hooks, continuous or discontinuous ridges, columns or annular shapes and can be regular or irregular, or any combination thereof. The overall adhesives to structured surface contact is also dependent on the adhesive thickness, rheology and structure. However, structure height, adhesive properties and structure distribution and shape should be such that the adhesive is in intermittent contact with the structured surface with interruptions in the adhesive to structured surface direct contact, on average, of about 20 to 80 per cm in the peel direction. This interruption in the adhesive to structured surface contact is provided by the valley portions between adjacent peaks.

The structured surface can be provided on any surface of a diaper, or like incontinent article, feminine hygiene article or any other surface where smooth peel performance is desired. In a diaper this would include release tapes or films, a water impermeable backsheet or a frontal reinforcement film or tape on the backsheet any of which could be either release coated or not release coated. A preferred release coated surface would be a coextruded thermoplastic polymer material with the outermost thermoplastic layer containing a melt additive release agent or a release agent modified polymer.

## Claims

1. A pressure-sensitive adhesive closure system comprising at least two opposing closure *surfaces* and a pressure-sensitive adhesive tape tab fastener removably attached to a first face of a first closure surface at a first free end of the tape tab fastener by a first pressure-sensitive adhesive layer portion on a first face of said tape tab free end, a second opposing end of said tape tab fastener permanently attached to a first face of a second opposing closure surface, wherein the first pressure-sensitive adhesive layer portion is comprised of a structured adhesive region the structured adhesive region having a continuous pressure-sensitive adhesive coating the continuous pressure-sensitive adhesive coating having adhesive peak structures creating adhesive contact regions at top portions of said adhesive peak structures, **characterized in that**, the cross-sectional area of said adhesive contact regions comprising less than 75 percent of the cross-sectional area of the structured adhesive region planar cross section with an average spacing distance between adjacent adhesive contact regions formed by adjacent adhesive peak structures being less than 500 microns the adhesive peak structures being adhesive peak structures with adjacent adhesive valley structures with an average peak to valley structure height of from 10 to 500 microns and said adhesive peaks having a width aspect ratio (WAR) of from 0.2 to 10.

2. The pressure-sensitive adhesive closure system of claim 1 wherein average peak to valley structure height is from 25 to 250 microns and the average spacing distance between adjacent adhesive contact regions is less than 500 microns.

3. The pressure-sensitive adhesive closure system of claim 1 or 2 wherein said first pressure-sensitive adhesive layer portion is a discontinuous adhesive coating and said adhesive peak structures have an average adhesive peak structure height of greater than 5 microns.

4. The pressure-sensitive adhesive closure system of any of claims 1 to 3 wherein said second opposing end of said tape tab is permanently attached to said second opposing closure surface by a second pressure-sensitive adhesive layer portion and said first and second pressure-sensitive adhesive layer portions are a continuous pressure-sensitive adhesive coating of the same adhesive on said first face of said tape tab.

5. The pressure-sensitive adhesive closure system of any of claims 1 to 4 further comprising a structured release liner, having a first structured face with a structured region of peak and valley irregularities, adjacent said second opposing closure surface wherein said structured adhesive region on said tape tab fastener first free end is in mating contact with said structured release liner, at least at the peak and valley structured region, prior to use, wherein the release liner peak structures have top portions and base portions.

6. The pressure-sensitive adhesive closure system of claim 5 wherein the structured release liner first structured face comprises peak structures having an average peak height greater than an average thickness of said first pressure-sensitive adhesive layer portion, the T-peel of said tape tab fastener first free end from said structured release liner first face being less than 120 Newton/meter.

7. The pressure-sensitive adhesive closure system of claim 6 wherein said structured release liner comprises a thermoplastic material with no release coating on the first structured face in contact with said first pressure-sensitive adhesive layer portion and less than 25 percent of a cross-sectional area, of a planar cross section parallel to said first structured face structured region, taken at the top portions of said release liner peak structures, contacts said first pressure-sensitive adhesive layer portion at said tape tab fastener first free end.

8. The pressure-sensitive adhesive closure system of claim 7 wherein said structured release liner comprises a structured release tape having a pressure-sensitive adhesive layer on a second face opposite said first structured face, said structured release tape second face adhered to said second opposing closure surface on a second face opposite said first face of said second opposing closure surface.

9. A diaper comprising the pressure-sensitive adhesive closure system of claim 8 wherein said first faces of said first and second opposing closure surfaces comprise an outer water impermeable surface of a diaper and said second opposite faces to said first faces comprise a water permeable inner layer of said diaper.

10. The pressure-sensitive adhesive closure system of claim 8 or 9 wherein said structured release tape first structured face comprises a series of upstanding stems and wherein said structured release tape upstanding stems have a peak top portion which continuously tapers to a base portion.

11. The pressure-sensitive adhesive closure system of any of claims 1 to 10 wherein each of said adhesive peak structures have a width aspect ratio (**WAR**) of from 1.0 to 5.

12. The pressure-sensitive adhesive closure system of any of claims 1 to 11 wherein each of said adhesive peak structures forms an adhesive contact region surrounding a central valley or caldera-like depression and the average height of said adhesive peak structures over said central valley is at least 5 microns.

13. The pressure-sensitive adhesive closure system of claim 12 wherein the average height of said adhesive peak structures over said central valley is at least 10 microns.

14. The pressure-sensitive adhesive closure system of claim 12 or 13 wherein said central valleys have an average width to depth ratio (WND) of from 1 to 100.

15. The pressure-sensitive adhesive closure system of claim 12 or 13 wherein said central valleys have an average width to depth ratio (WND) of from 5 to 75.

16. The pressure-sensitive adhesive closure system of any of claims 1 to 15 wherein said adhesive peak structures taper from said peak structure top portions to said peak structure base portions at an angle of from 10 to 60 degrees.

17. The pressure-sensitive adhesive closure system of claim 16 wherein said adhesive peak structures comprise longitudinally extending ridges forming center groove valleys.

18. The pressure-sensitive adhesive closure system of any of claims 12 to 15 wherein said adhesive peak structures comprise a flat plateau surrounding at least one depression.

19. The pressure-sensitive adhesive closure system of any of claims 1 to 18 wherein the pressure-sensitive adhesive of said first pressure-sensitive adhesive layer portion comprises a tackified A-B block copolymer elastomer wherein the A block comprises a monoalkenyl arene and the B block is an elastomeric conjugated diene.

20. The pressure-sensitive adhesive closure system of claim 19 wherein said pressure-sensitive adhesive comprises a tackified A-B type block copolymer elastomer wherein the A block is crosslinked.

## Patentansprüche

1. Druckempfindliches Klebeverschlußsystem mit mindestens zwei entgegengesetzten Verschlußflächen und einem druckempfindlichen Klebebandstreifen-Befestigungselement, das durch einen ersten druckempfindlichen Klebeschichtabschnitt an einer ersten Fläche des freien Endes des Bandstreifens abnehmbar an einer ersten Fläche einer ersten Verschlußfläche an einem ersten freien Ende des Bandstreifen-Befestigungselements angebracht ist, wobei ein zweites entgegengesetztes Ende des Bandstreifen-Befestigungselements permanent an einer ersten Fläche einer zweiten entgegengesetzten Verschlußfläche angebracht ist, wobei der erste druckempfindliche Klebeschichtabschnitt aus einem strukturierten Klebebereich besteht, wobei der strukturierte Klebebereich eine zusammenhängende druckempfindliche Klebebeschichtung aufweist, wobei die zusammenhängende druckempfindliche Klebebeschichtung Klebstoffspitzen-Strukturen aufweist, die an den oberen Abschnitten der Klebstoffspitzen-Strukturen Klebstoff-Kontaktbereiche bilden, **dadurch gekennzeichnet, daß** die Querschnittsfläche der Klebstoff-Kontaktbereiche weniger als 75 Prozent der Querschnittsfläche des ebenen Querschnitts aufweist, wobei der durchschnittliche Abstand zwischen durch benachbarte Klebstoffspitzen-Strukturen gebildeten benachbarten Klebstoff-Kontaktbereichen weniger als 500 Mikrometer beträgt, wobei die Klebstoffspitzen-Strukturen benachbarte Klebstofftal-Strukturen mit einer durchschnittlichen Strukturhöhe von Spitze zu Tal von 10 bis 500 Mikrometer sind und wobei die Klebstoffspitzen ein Breiten-Seitenverhältnis (WAR) von 0,2 bis 10 aufweisen.

2. Druckempfindliches Klebeverschlußsystem nach Anspruch 1, wobei die durchschnittliche Strukturhöhe von Spitze zu Tal von 25 bis 250 Mikrometer reicht und der durchschnittliche Abstand zwischen benachbarten Klebstoff-Kontaktbereichen weniger als 500 Mikrometer beträgt.

3. Druckempfindliches Klebeverschlußsystem nach Anspruch 1 oder 2, wobei der erste druckempfindliche Klebeschichtabschnitt eine unzusammenhängende Klebstoffbeschichtung ist und die Klebstoffspitzen-Strukturen eine durchschnittliche Höhe von mehr als 5 Mikrometer aufweisen.

4. Druckempfindliches Klebeverschlußsystem nach einem der Ansprüche 1 bis 3, wobei das zweite entgegengesetzte Ende des Bandstreifens durch einen zweiten druckempfindlichen Klebeschichtabschnitt permanent an der zweiten entgegengesetzten Verschlußfläche angebracht ist und wobei der erste und der zweite druckempfindliche Klebeschichtabschnitt eine zusammenhängende druckempfindliche Klebstoffbeschichtung des gleichen Klebstoffs auf der ersten Fläche des Bandstreifens bilden.

5. Druckempfindliches Klebeverschlußsystem nach einem der Ansprüche 1 bis 4, welches weiter ein strukturiertes ablösbares Kaschierpapier aufweist, das neben der zweiten entgegengesetzten Verschlußfläche eine erste strukturierte Fläche mit einem strukturierten Bereich von Unregelmäßigkeiten aus Spitzen und Tälern aufweist, wobei der strukturierte Klebstoffbereich auf dem ersten freien Ende des Bandstreifen-Befestigungselements vor der Verwendung zumindest am strukturierten Bereich der Spitzen und Täler in übereinstimmendem Kontakt mit dem strukturierten ablösbaren Kaschierpapier steht, wobei die Spitzenstrukturen des ablösbaren Kaschierpapiers obere Abschnitte und Sockelabschnitte aufweisen.

6. Druckempfindliches Klebeverschlußsystem nach Anspruch 5, wobei die erste strukturierte Außenfläche des strukturierten ablösbaren Kaschierpapiers Spitzenstrukturen aufweist, die eine durchschnittliche Spitzenhöhe haben, die größer ist als die durchschnittliche Dicke des ersten druckempfindlichen Klebeschichtabschnitts, wobei der T-Abziehwert des ersten freien Endes des Bandstreifen-Befestigungselements gegenüber der ersten Fläche des strukturierten ablösbaren Kaschierpapiers kleiner als 120 Newton/Meter beträgt.

7. Druckempfindliches Klebeverschlußsystem nach Anspruch 6, wobei das strukturierte ablösbare Kaschierpapier ein thermoplastisches Material aufweist, wobei an der ersten strukturierten Außenfläche keine Ablösebeschichtung in Kontakt mit dem ersten druckempfindlichen Klebeschichtabschnitt steht und weniger als 25 Prozent der Querschnittsfläche eines ebenen Querschnitts parallel zum strukturierten Bereich der ersten strukturierten Fläche an den oberen Abschnitten der Spitzenstrukturen des ablösbaren Kaschierpapiers den ersten druckempfindlichen Klebeschichtabschnitt am ersten freien Ende des Bandstreifen-Befestigungselements berühren.

8. Druckempfindliches Klebeverschlußsystem nach Anspruch 7, wobei das strukturierte ablösbare Kaschierpapier ein strukturiertes Ablöseband mit einer druckempfindlichen Klebeschicht an einer der ersten strukturierten Fläche entgegengesetzten zweiten Fläche aufweist, wobei die zweite Fläche des strukturierten Ablösebands an einer der ersten Fläche der zweiten entgegengesetzten Verschlußfläche entgegengesetzten zweiten Fläche an der zweiten entgegengesetzten Verschlußfläche haftet.

9. Windel mit dem druckempfindlichen Klebeverschlußsystem nach Anspruch 8, wobei die ersten Flächen der ersten und der zweiten entgegengesetzten Verschlußflächen eine äußere wasserundurchlässige Fläche einer Windel aufweisen und die den ersten Flächen entgegengesetzten zweiten Flächen eine wasserdurchlässige Innenschicht der Windel aufweisen.

10. Druckempfindliches Klebeverschlußsystem nach Anspruch 8 oder 9, wobei die erste strukturierte Fläche des strukturierten Ablösebands eine Reihe hochstehender Stiele aufweist und wobei die hochstehenden Stiele des strukturierten Ablösebands einen oberen Spitzenabschnitt aufweisen, der sich kontinuierlich zu einem Basisabschnitt verjüngt.

11. Druckempfindliches Klebeverschlußsystem nach einem der Ansprüche 1 bis 10, wobei jede der Klebstoffspitzen-Strukturen ein Breiten-Seitenverhältnis (WAR) von 1,0 bis 5 aufweist.

12. Druckempfindliches Klebeverschlußsystem nach einem der Ansprüche 1 bis 11, wobei jede der Klebstoffspitzen-Strukturen einen Klebstoff-Kontaktbereich bildet, der eine mittlere tal- oder kraterartige Vertiefung umgibt, und wobei die durchschnittliche Höhe der Klebstoffspitzen-Strukturen über dem mittleren Tal mindestens 5 Mikrometer beträgt.

13. Druckempfindliches Klebeverschlußsystem nach Anspruch 12, wobei die durchschnittliche Höhe der Klebstoffspitzen-Strukturen über dem mittleren Tal mindestens 10 Mikrometer beträgt.

14. Druckempfindliches Klebeverschlußsystem nach Anspruch 12 oder 13, wobei die mittleren Täler ein durchschnittliches Breite-zu-Tiefe-Verhältnis (WND) von 1 bis 100 aufweisen.

15. Druckempfindliches Klebeverschlußsystem nach Anspruch 12 oder 13, wobei die mittleren Täler ein durchschnittliches Breite-zu-Tiefe-Verhältnis (WND) von 5 bis 75 aufweisen.

16. Druckempfindliches Klebeverschlußsystem nach einem der Ansprüche 1 bis 15, wobei sich die Klebstoffspitzen-Strukturen von den oberen Abschnitten der Spitzenstruktur zu den Basisabschnitten der Spitzenstruktur unter einem Winkel von 10 bis 60 Grad verjüngen.

17. Druckempfindliches Klebeverschlußsystem nach Anspruch 16, wobei die Klebstoffspitzen-Strukturen sich in Längsrichtung erstreckende Grate aufweisen, die mittlere Nutentäler bilden.

18. Druckempfindliches Klebeverschlußsystem nach einem der Ansprüche 12 bis 15, wobei die Klebstoffspitzen-Strukturen ein ebenes Plateau aufweisen, das mindestens eine Vertiefung umgibt.

19. Druckempfindliches Klebeverschlußsystem nach einem der Ansprüche 1 bis 18, wobei der druckempfindliche Klebstoff des ersten druckempfindlichen Klebeschichtabschnitts ein klebrig gemachtes A-B-Blockcopolymerelastomer aufweist, wobei der A-Block ein Monoalkenylaren aufweist und der B-Block ein elastomeres konjugiertes Dien ist.

20. Druckempfindliches Klebeverschlußsystem nach Anspruch 19, wobei der druckempfindliche Klebstoff ein klebrig gemachtes A-B-Blockcopolymerelastomer aufweist, wobei der A-Block vernetzt ist.

## Revendications

1. Système de fermeture d'adhésif sensible à la pression comprenant au moins deux surfaces de fermeture opposées et un élément de fixation de la forme d'une patte de ruban d'adhésif sensible à la pression attaché de façon détachable à une première face d'une première surface de fermeture à une première extrémité libre de l'élément de fixation sous forme de patte de ruban par une première partie de couche d'adhésif sensible à la pression sur une première face de ladite extrémité libre de patte de ruban, et une seconde extrémité opposée dudit élément de fixation sous forme de patte de ruban attachée de façon permanente à une première face d'une seconde surface de fermeture opposée, dans lequel la première partie de couche d'adhésif sensible à la pression est formée d'une zone d'adhésif structurée, la zone d'adhésif structurée comportant un revêtement d'adhésif sensible à la pression continu, le revêtement d'adhésif sensible à la pression continu comportant des structures de pic d'adhésif formant des zones de contact d'adhésif à des parties supérieures desdites structures de pic d'adhésif, **caractérisé en ce que** l'aire en coupe transversale desdites zones de contact d'adhésif constitue moins de 75 % de l'aire en coupe transversale de la section transversale plane de zone d'adhésif structurée avec une distance d'espacement moyenne entre zones de contact d'adhésif adjacentes formées par des structures de pic d'adhésif adjacentes qui est inférieure à 500 microns, les structures de pic d'adhésif étant des structures de pic d'adhésif avec des structures de vallée d'adhésif adjacentes avec une hauteur de structure de pic à vallée moyenne de 10 à 500 microns et lesdits pics d'adhésif ayant un rapport d'aspect en largeur (WAR) de 0,2 à 10.

2. Système de fermeture d'adhésif sensible à la pression suivant la revendication 1, dans lequel la hauteur de structure de pic à vallée moyenne est de 25 à 250 microns et la distance d'espacement moyenne entre zones de contact d'adhésif adjacentes est inférieure à 500 microns.

3. Système de fermeture d'adhésif sensible à la pression suivant l'une ou l'autre des revendications 1 et 2, dans lequel la première partie de couche d'adhésif sensible à la pression est un revêtement d'adhésif discontinu et les structures de pic d'adhésif précitées ont une hauteur de structure de pic d'adhésif moyenne supérieure à 5 microns.

4. Système de fermeture d'adhésif sensible à la pression suivant l'une quelconque des revendications 1 à 3, dans lequel la seconde extrémité opposée précitée de la patte de ruban précitée est attachée de façon permanente à la seconde surface de fermeture opposée précitée par une seconde partie de couche d'adhésif sensible à la pression et lesdites première et seconde parties de couche d'adhésif sensible à la pression constituent un revêtement d'adhésif sensible à la pression continu du même adhésif sur la première surface de ladite patte de ruban.

5. Système de fermeture d'adhésif sensible à la pression suivant l'une quelconque des revendications 1 à 4, comprenant de plus une bande de séparation structurée, comportant une première face structurée avec une zone structurée d'irrégularités de pic et de vallée. adjacente à la seconde surface de fermeture opposée précitée, dans lequel ladite zone d'adhésif structurée sur la première extrémité libre de l'élément de fixation sous forme de patte de ruban précitée est en contact d'appariement avec la bande de séparation structurée précitée, au moins à la zone structurée en pic et vallée, avant utilisation, dans lequel les structures de pic de la bande de séparation comportent des parties supérieures et des parties de base.

6. Système de fermeture d'adhésif sensible à la pression suivant la revendication 5, dans lequel la première face structurée de la bande de séparation structurée comprend des structures de pic ayant une hauteur de pic moyenne supérieure à une épaisseur moyenne de la première partie de couche d'adhésif sensible à la pression précitée, le pelage en T de la première extrémité libre de l'élément de fixation en forme de patte de ruban précitée de la première face de la bande de séparation structurée étant inférieur à 120 Newtons/mètre.

7. Système de fermeture d'adhésif sensible à la pression suivant la revendication 6, dans lequel la bande de séparation structurée précitée comprend une matière thermoplastique sans revêtement de séparation sur la première face structurée en contact avec la première partie de couche d'adhésif sensible à la pression précitée et moins de 25 % d'une aire en coupe transversale, d'une section transversale plane parallèle à la zone structurée de la première face structurée précitée, prise aux parties supérieures des structures de pic de bande de séparation précitées, sont au contact de la première partie de couche d'adhésif sensible à la pression à la première extrémité libre de l'élément de fixation sous forme de patte de ruban précitée.

8. Système de fermeture d'adhésif sensible à la pression suivant la revendication 7, dans lequel la bande de séparation structurée précitée comprend un ruban de séparation structuré comportant une couche d'adhésif sensible à la pression sur une seconde face opposée à la première face structurée précitée, ladite seconde face de ruban de séparation structurée adhérant à la seconde surface de fermeture opposée sur une seconde face opposée à ladite première face de la seconde surface de fermeture opposée précitée.

9. Lange comprenant le système de fermeture d'adhésif sensible à la pression suivant la revendication 8, dans lequel les premières faces précitées de la première et de la seconde surfaces de fermeture opposées comprennent une surface imperméable à l'eau extérieure d'un lange et lesdites secondes faces opposées auxdites premières faces comprennent une couche intérieure perméable à l'eau du lange précité.

10. Système de fermeture d'adhésif sensible à la pression suivant l'une ou l'autre des revendications 8 et 9, dans lequel la première face structurée du ruban de séparation structuré précitée comprend une série d'émergences droites et dans lequel lesdites émergences droites du ruban de séparation structuré comportent une partie supérieure de pic qui s'amincit de façon continue jusqu'à une partie de base.

11. Système de fermeture d'adhésif sensible à la pression suivant l'une quelconque des revendications 1 à 10, dans lequel chacune des structures de pic d'adhésif précitées a un rapport d'aspect en largeur (WAR) de 1,0 à 5.

12. Système de fermeture d'adhésif sensible à la pression suivant l'une quelconque des revendications 1 à 11, dans lequel chacune des structures de pic d'adhésif précitées forme une zone de contact d'adhésif entourant une vallée centrale ou dépression du type caldeira et la hauteur moyenne desdites structures de pic d'adhésif par rapport à la vallée centrale est d'au moins 5 microns.

13. Système de fermeture d'adhésif sensible à la pression suivant la revendication 12, dans lequel la hauteur moyenne des structures de pic d'adhésif précitées par rapport à la vallée centrale précitée est d'au moins 10 microns.

14. Système de fermeture d'adhésif sensible à la pression suivant l'une ou l'autre des revendications 12 et 13, dans lequel les vallées centrales précitées ont un rapport largeur à profondeur moyen (WND) de 1 à 100.

15. Système de fermeture d'adhésif sensible à la pression suivant l'une ou l'autre des revendications 12 et 13, dans lequel les vallées centrales précitées ont un rapport largeur à profondeur moyen (WND) de 5 à 75.

16. Système de fermeture d'adhésif sensible à la pression suivant l'une quelconque des revendications 1 à 15, dans lequel les structures de pic d'adhésif précitées vont en s'amincissant des parties supérieures de structure de pic aux parties de base de structure de pic suivant un angle de 10 à 60 degrés.

17. Système de fermeture d'adhésif sensible à la pression suivant la revendication 16, dans lequel lesdites structures de pic d'adhésif comprennent des crêtes s'étendant longitudinalement formant des vallées en forme de rainure centrales.

18. Système de fermeture d'adhésif sensible à la pression suivant l'une quelconque des revendications 12 à 15, dans lequel les structures de pic d'adhésif précitées comprennent un plateau plat entourant au moins une dépression.

19. Système de fermeture d'adhésif sensible à la pression suivant l'une quelconque des revendications 1 à 18, dans lequel l'adhésif sensible à la pression de la première partie de couche d'adhésif sensible à la pression comprend un élastomère de copolymère bloc A-B collé dans lequel le bloc A comprend un monoalcényl arène et le bloc B est un diène conjugué élastomère.

20. Système de fermeture d'adhésif sensible à la pression suivant la revendication 19, dans lequel ledit adhésif sensible à la pression comprend un élastomère de copolymère bloc du type A-B collé dans lequel le bloc A est réticulé.
